# EUROPEAN PATENT SPECIFICATION

(11) **EP 3 443 349 B1**
(45) Date of publication and mention of the grant of the patent: **10.06.2020**
(21) Application number: 17724632.9
(22) Date of filing: 10.04.2017
(51) Int. Cl.: G01N 33/564, G01N 33/574

(54) **MARKERS FOR DIAGNOSTIC AND PROGNOSTIC OF OVARIAN CANCER**
NEUE DIAGNOSTISCHE UND PROGNOSTISCHE MARKER FÜR OVARIALKARZINOM
NOUVEAUX MARQUEURS DE DIAGNOSTIC ET DE PRONOSTIC DU CANCER DE L'OVAIRE

(30) Priority: 11.04.2016 IT UA20162478
(43) Date of publication of application: 20.02.2019
(73) Proprietor: Molipharma S.r.l., 86100 Campobasso (IT)
(72) Inventor: SCAMBIA, Giovanni, 86100 Campobasso (IT); PETRILLO, Marco, 86100 Campobasso (IT); GALLO, Daniela, 86100 Campobasso (IT); MARTINELLI, Enrica, 86100 Campobasso (IT); FANELLI, Mara, 86100 Campobasso (IT); BATTAGLIA, Alessandra, 86100 Campobasso (IT); FATTOROSSI, Andrea, 86100 Campobasso (IT); RASPAGLIO, Giuseppina, 86100 Campobasso (IT)
(74) Representative: Predazzi, Valentina
(86) International application number: PCT/IB2017/052048
(87) International publication number: WO 2017/178948

(56) References cited:
- US-A1- 2004 191 841
- MOU Z ET AL: "Immunoproteomics to identify tumor-associated antigens eliciting humoral response", CANCER LETTERS, NEW YORK, NY, US, vol. 278, no. 2, 18 June 2009 (2009-06-18) , pages 123-129, XP026046102, ISSN: 0304-3835, DOI: 10.1016/J.CANLET.2008.09.009 [retrieved on 2008-10-17]
- RAMILA PHILIP ET AL: "Shared Immunoproteome for Ovarian Cancer Diagnostics and Immunotherapy: Potential Theranostic Approach to Cancer", JOURNAL OF PROTEOME RESEARCH., vol. 6, no. 7, 5 June 2007 (2007-06-05), pages 2509-2517, XP055320062, US ISSN: 1535-3893, DOI: 10.1021/pr0606777
- JINTONG DU ET AL: "Overexpression of Class III beta-tubulin, Sox2, and nuclear Survivin is predictive of taxane resistance in patients with stage III ovarian epithelial cancer", BMC CANCER, BIOMED CENTRAL, LONDON, GB, vol. 15, no. 1, 23 July 2015 (2015-07-23), page 536, XP021225916, ISSN: 1471-2407, DOI: 10.1186/S12885-015-1553-X
- GAO SONG ET AL: "Clinical implications of REST and TUBB3 in ovarian cancer and its relationship to paclitaxel resistance", TUMOR BIOLOGY, KARGER, BASEL, CH, vol. 33, no. 5, 10 June 2012 (2012-06-10), pages 1759-1765, XP035966966, ISSN: 1010-4283, DOI: 10.1007/S13277-012-0435-Y [retrieved on 2012-06-10]
- G. FERRANDINA: "Class III -Tubulin Overexpression Is a Marker of Poor Clinical Outcome in Advanced Ovarian Cancer Patients", CLINICAL CANCER RESEARCH, vol. 12, no. 9, 1 May 2006 (2006-05-01), pages 2774-2779, XP055320558, US ISSN: 1078-0432, DOI: 10.1158/1078-0432.CCR-05-2715 cited in the application

## Description

The present invention relates to the identification of new diagnostic and prognostic markers for ovarian cancer in humans, use of kit comprising them, their use and new diagnostic and/or prognostic methods for the identification of ovarian cancer in a human being.

### PRIOR ART

Ovarian carcinoma is the first cause of death among gynecologic tumours. Owing to the high growth rate and invasive ability of ovarian carcinoma, most patients present an advanced-stage disease at diagnosis. Moreover, despite cytoreductive surgery progresses and the introduction of carboplatin/paclitaxel combinations as a conventional therapeutic regimen, intrinsic or acquired drug resistance remains the main determinant of chemotherapy failure and, as a result, of a fatal clinical outcome.

The improvement in the understanding of ovarian carcinoma-associated genetics enabled to develop therapies with molecular targets. Recently, in ovarian cancer a series of tumour-associated antigens have been identified which play a relevant role in tumour angiogenesis, in proliferation and in the metastasizing process. Among these antigens, the best characterized ones are vascular endothelial growth factor (VEGF), basic fibroblast growth factor (bFGF), alpha-folate receptor (αFr) and members of the epidermal growth factor receptor family.

Microtubules are intracellular structures important for many cellular processes, such as mitosis, motility and intracellular transportation. They are comprised of alpha- and beta-tubulin monomers, which spontaneously assemble to form heterodimers. Class III tubulin (TUBB3), encoded by the TUBB3 gene, is a protein constitutively expressed in human neuronal cells in the central and peripheral nervous system and in Sertoli cells of the seminiferous tubules of the testis (Mariani M, Karki R, Spennato M, Pandya D, He S, Andreoli M, Fiedler P, Ferlini C. Class III β-tubulin in normal and cancer tissues. Gene. 2015 Jun 1;563(2):109-14). This protein is not normally expressed in epithelial cells, whereas it is overexpressed in many types of carcinoma, such as the lung, stomach, prostatic and ovarian ones (Mariani M.2015; Ferrandina G, Zannoni GF, Martinelli E, Paglia A, Gallotta V, et al. Class III beta-tubulin overexpression is a marker of poor clinical outcome in advanced ovarian cancer patients. Clin Cancer Res. 2006 May 1;12(9):2774-9). Recent literature data document by immunohistochemical methods that TUBB3 overexpression in lung, prostatic, stomach and ovarian cancer is associated with unfavourable prognosis (Mozzetti S, Ferlini C, Concolino P, Filippetti F, Raspaglio G, Prislei S, et al. Class III beta tubulin overexpression is a prominent mechanism of paclitaxel resistance in ovarian cancer. Clin Cancer Res. 2005 Jan 1;11(1):298-305; Ferrandina G 2006; Kavallaris M. Microtubules and resistance to tubulin-binding agents. Nat Rev Cancer. 2010 Mar;10(3):194-204), whereas there is no correlation between TUBB3 overexpression and aggressiveness in other tumours, such as cervical ones (Ferrandina G, Martinelli E, Zannoni GF, Distefano M et al. Expression of class III β tubulin in cervical cancer patients administered preoperative radiochemotherapy: Correlation with response to treatment and clinical outcome. Gynecology Oncology 2007. 326-330). TUBB3 role remains controversial in other tumours, such as, e.g., breast carcinoma, in which hormonal influence plays a key role in TUBB3 overexpression (Mariani M 2015).

In the initial stage of cancer development and during its progression, mutations are produced which lead to protein overexpression, or to expression of ectopic proteins that, in case of cellular injury, can be exposed and, when recognized by the immune system, induce production of specific antibodies. In fact, in the last years many studies demonstrated the presence of autoantibodies against tumour-associated antigens (TAA) in the blood of cancer patients. These tumour-specific autoantibodies, also thanks to their biochemical characteristics of stability and high concentration in serum, can easily be identified and, therefore, can be considered reliable biomarkers in the early diagnosis of a tumour, such as esophageal, lung, ovarian, colorectal carcinomas and melanoma (Defresne F, Bouzin C, Guilbaud C, Dieu M, Delaive E, Michiels C, et al. Differential influence of anticancer treatment and angiogenesis on the seric titer of autoantibody used as tumour and metastasis biomarker. Neoplasia; 2010.vo112 July, 565-570; Grassadonia A, Tinari N, Natoli C, Yahalom G, lacobelli S. Circulating autoantibodies to LGALS3BP: a novel biomarker for cancer. Dis Markers. 2013;35(6):747-52).

Moreover, in numerous studies it has been demonstrated that the presence of autoantibodies specific against TAA is associated with a greater probability of recurrence; therefore, the dosage of anti-TAA autoantibodies can constitute a valid support in laboratory diagnosis associated with assessment of tumour aggressiveness.

Bansal D et al, in 2009 (Bansal D, Herbert F, Lim P, Deshpande P, Bécavin C, Guiyedi V, et al. IgG autoantibody to brain beta tubulin III associated with cytokine cluster-II discriminate cerebral malaria in central India. PLoS One. 2009 Dec 14;4(12):e8245.) demonstrated (by ELISA, enzyme-linked immunosorbent assay, method) the presence of IgG-class anti-TUBB3 autoantibodies in patients affected by malaria caused by *Plasmodium falciparum* parasite, where there is nervous cell destruction. In this work, the authors do not specify the production and purification characteristics of the TUBB3 recombinant protein utilized in the ELISA assay.

As already mentioned above, many studies on the overexpression and prognostic value of TUBB3, analysed by immunochemistry, in ovarian carcinoma have been published. However, it emerged that, given ovarian carcinoma heterogeneity, and considering that tumour cells subjected to limiting environmental stimuli (such as lack of oxygen and nutrients) activate survival "pathways" in which TUBB3 expression represents a key event, immunohistochemistry as a technique does not suffice to quantitate TUBB3 expression. In fact, TUBB3 expression varies in different samples of the same tumour.

US patent application US 2004/191841 discloses an *in vitro* method for the diagnosis of neuroblastoma in a patient by detecting the presence of autoantibodies specific for TUBB3. Mou Z et al., in 2009 (Mou Z et al. Immunoproteomics to identify tumor-associated antigens eliciting humoral response. Cancer letters. 2009 Jun 18; 278(2):123-129) discloses the identification of anti-S100A7 autoantibodies in serum samples from patients suffering from ovarian cancer.

Moreover, the assessment of TUBB3 expression using immunohistochemistry as a method requires:
- an invasive intervention for bioptic sample collection;
- the intervention of several professionals, such as technicians, biologists specialized in histopathology, and pathologists qualified and expert in the tissue to be studied;
- a relevant commitment in terms of time, as the various biopsies to be studied are first fixed, then processed and finally embedded, before being used for analysis;
- a high economical commitment, given the number of highly specialized persons involved and the materials consumed.

Therefore, new methods of diagnosis and prognosis of ovarian carcinoma, whose results be interpretable in a more objective and not operator-related manner, and not requiring bioptic sample collection from patients, are desirable.

### SUMMARY OF THE INVENTION

The Authors of the present invention discovered that intracellular presence of TUBB3 in ovarian tumour generates anyhow serum anti TUBB3 autoantibodies, and that, moreover, the concentration of these autoantibodies is related with disease prognosis.

The Inventors therefore set up a diagnostic and/or prognostic method, based on the detection of these autoantibodies in patients' sera, which leads to the overcoming of technical problems typical of the detection of TUBB3 presence in tumour tissue by immunohistochemistry techniques already discussed in the background of the invention. Specifically, the method disclosed in the present description is easy, quick, does not require high costs and employs for its carrying out a smaller number of professionals than the immunohistochemical technique. The Authors of the present invention demonstrated that patients affected by ovarian carcinoma experience high serum levels of anti-TUBB3 autoantibodies. On the contrary, in healthy subjects the serum levels of these autoantibodies are below the dosage determination limit.

One of the main advantages of the method of the invention lies in that this methodology does not require a significant preparation of the sample to be analysed and uses a few microliters of serum obtainable with the simple maneuver of collecting peripheral blood from patients (which, moreover, can be repeated several times during the follow-up of the patient).

Moreover, since the method of the present invention is based on the measuring of anti-TUBB3 autoantibodies in the serum with instrumental methodology, it enables an objective evaluation of analyte presence in the serum, evaluation which does not depend on the operator. Moreover, advantageously, the methods of the invention are suitable to guarantee the diagnosis of ovarian carcinoma and/or provide predictive information on drug resistance (chemoresistance), or lack thereof, of the same at an early stage.

Therefore, the invention relates to:
- an *in vitro* method for the diagnosis of ovarian carcinoma in a patient, comprising the following steps:
   a) determining the concentration C of anti-TUBB3 autoantibodies in a serum sample of said patient;
   b) comparing said concentration C of autoantibodies to a threshold value *x*, wherein a concentration of said autoantibodies higher than said threshold value identifies the presence of ovarian carcinoma in said patient;
- an *in vitro* method for the diagnosis of ovarian carcinoma and the prognosis of overall survival expectancy in case of presence of said ovarian carcinoma in a patient, comprising the following steps:
   a) determining the concentration C of anti-TUBB3 autoantibodies in a serum sample of said patient;
   b) comparing said concentration C of autoantibodies to a threshold value *x,* wherein a concentration of said autoantibodies higher than said threshold value *x* identifies the presence of ovarian carcinoma in said patient; and
   c) when said patient is positive to ovarian carcinoma, comparing said concentration C of autoantibodies to a threshold value y; wherein an increasing difference between said concentration C and said second threshold value *y* corresponds to a decreasing overall survival expectancy of said patient;
- *in vitro* methods for the prognosis of overall survival expectancy comprising the steps b) and c) of the above-reported methods on patients affected by ovarian carcinoma;
- use of kit for the detection of anti-TUBB3 autoantibodies in the carrying out of the above-reported methods.

### DETAILED DESCRIPTION OF THE FIGURES

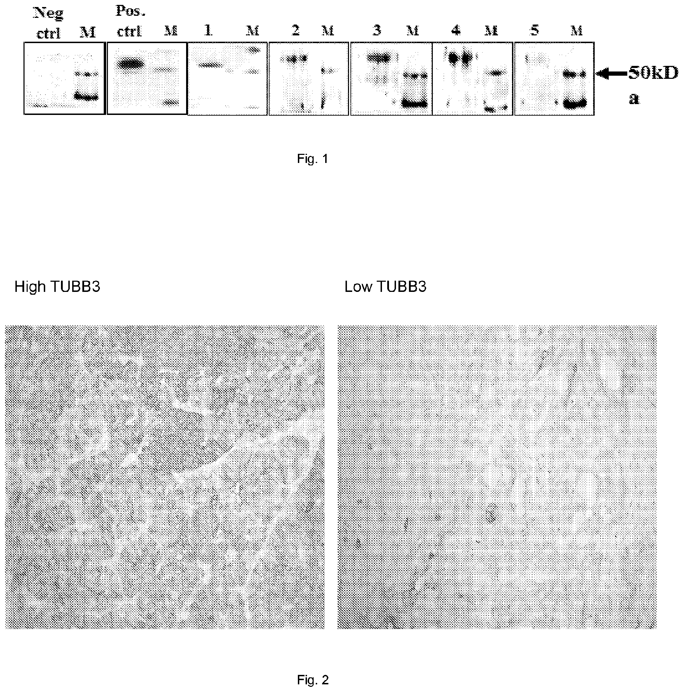

Figure 1: Western blot analysis for the detecting of anti-TUBB3 autoantibodies in sera of samples collected from patients with a diagnosis of ovarian carcinoma. Lanes 1 to 5 in the figure show the presence of anti-TUBB3 autoantibodies in sera of patients positive to anti-TUBB3 autoantibodies by ELISA method. All samples exhibit a marked reactivity with the recombinant TUBB3 protein loaded on the membrane. M denotes a chemoluminescent molecular marker. Negative control (neg ctrl) was made using the serum of a healthy donor, whereas positive control (pos ctrl) was made using an anti-TUBB3 polyclonal antibody.
Figure 2: Immunohistochemistry images representative of TUBB3 positivity on ovarian carcinoma. An example of high and low expression of TUBB3 protein in primary ovarian carcinoma is shown. TUBB3-specific immunostaining was exclusively limited to the cytoplasm.

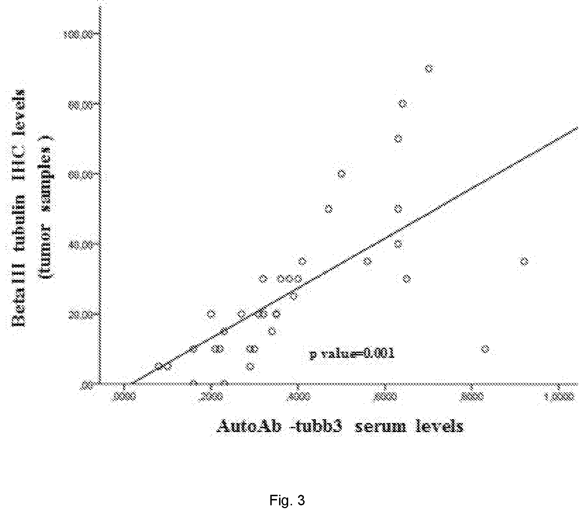

Figure 3. Correlation between TUBB3 protein levels in tumour samples and presence of anti-TUBB3 autoantibodies in the serum. In said figure, by using Spearman nonparametric statistical test, it is highlighted how a linear and statistically significant correlation exists among TUBB3 levels assessed in the tumour by immunohistochemical analysis (*y* axis) and anti-TUBB3 autoantibodies levels in the serum assessed by ELISA (*x* axis) in the investigated cohort of 49 patients affected by ovarian carcinoma. The correlation between the two parameters appears significant, with a very favourable Spearman correlation coefficient, equal to 0.804.

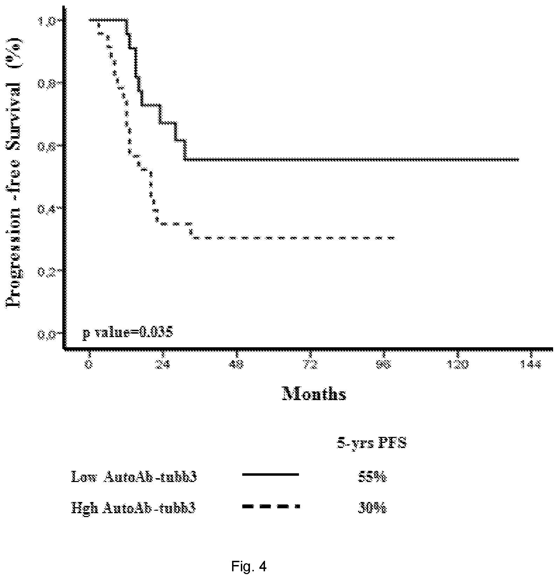

Figure 4: Kaplan-Meier analysis of progression-free survival (PFS) in patients affected by ovarian carcinoma. PFS curve in patients with ovarian carcinoma depending on the concentration of anti-TUBB3 autoantibodies (in the present description, and in the figures, also referred to as auto-AbTUBB3). Continuous line: low levels of anti-TUBB3 autoantibodies; broken line: high levels of anti-TUBB3 autoantibodies.

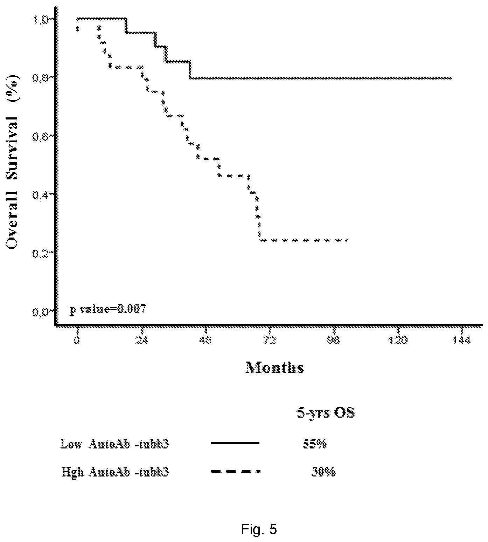

Figure 5: Kaplan-Meier analysis of overall survival (OS) in patients affected by ovarian carcinoma. OS curve in patients with ovarian carcinoma depending on the concentration of anti-TUBB3 autoantibodies (also indicated in the present description and in the figures as auto-AbTUBB3). Continuous line: low levels of anti-TUBB3 autoantibodies; broken line: high levels of anti-TUBB3 autoantibodies.

### DETAILED DESCRIPTION OF THE INVENTION

As already described above, the present invention provides new diagnostic and/or prognostic methods (relative to OS and/or drug resistance to taxol and/or derivatives thereof) in the field of oncological diagnostics, that can be carried out on serum samples.

Such methods need no bioptic sample collection, can be carried out quickly and are easy to perform. They do not require a subjective evaluation of the result, for which competences consolidated over time need to be demonstrated, but rather an objective (instrumental) assessment.

As to diagnostics, the methods of the invention enable a rapid and very reliable diagnosis about the presence or absence of ovarian carcinoma, and in a non-invasive manner, since a sample of peripheral blood from the patient suffices to perform the dosage (assay). Moreover, the methods of the present invention enable to acquire in very quick times information of remarkable relevance for patients affected by ovarian carcinoma, about carcinoma aggressiveness and about the possibility that it be resistant to the class of chemotherapeutical agents represented by taxol and/or derivatives thereof, normally utilized in first-line treatment for ovarian carcinoma.

In fact, the prognostic value of the methods of the present invention enables the medical team that follows the patient to immediately proceed with the most advisable therapeutic treatment, avoiding wasting precious time in cures that have scarce probabilities of success and, as an at least equally relevant fact, preserving the patient from probably useless side effects associated with ineffective therapies.

Since in patients affected by ovarian carcinoma drug resistance to taxol and/or derivatives thereof is normally associated with an overall survival (also referred to as OS in the present description) markedly lower than the OS observed in treatment-sensitive patients, evidently the existence of *in vitro* methods enabling an early estimate of individual survival, and a quick assessment of drug resistance in the individual patient, enables to make a personalized therapeutic program with remarkable benefit to the patient and also to the National health system.

An object of the present invention is represented by an *in vitro* method for the diagnosis of ovarian carcinoma in a patient, comprising the following steps:
a) determining the concentration C of anti-TUBB3 autoantibodies in a serum sample;
b) comparing the concentration C of autoantibodies with a threshold value *x,* wherein a concentration of said autoantibodies higher than said threshold value identifies the presence of ovarian carcinoma in said patient.

The threshold value *x,* for the purposes of the present description, may be any threshold value extrapolated from a cohort of samples comprising healthy women and women affected by ovarian carcinoma.

Said threshold value x can therefore be calculated by comparing distribution curves of serum concentrations of anti-TUBB3 autoantibodies of samples collected from healthy individuals and distribution curves of serum concentrations of anti-TUBB3 autoantibodies of samples collected from patients affected by ovarian carcinoma, and using a ROC curve for defining said threshold value according to the desired specificity and sensitivity.

According to one embodiment, the threshold value between pathological and normal levels of anti-TUBB3 autoantibodies can be extrapolated from a ROC curve. In practice, said analysis compares distribution curves of the seric (serum) levels of anti-TUBB3 antibodies in healthy subjects and in diseased subjects, analyses the degree of overlapping of the two distribution curves, and enables to calculate a threshold level (cut-off) which minimizes false-positive and false-negative mistakes.

The threshold value can be established, inside this curve, based on sensitivity and desired specificity. The threshold value obtained through ROC curve analysis is able to discern, e.g., a set of healthy subjects from a set of pathology-affected subjects, by analysing the area subtended by the ROC curve (Area Under Curve, AUC). This is equivalent to the probability that the result of the test carried out on one individual randomly extracted from the group of diseased subjects be higher than that of one randomly extracted from the group of healthy subjects.

For the purposes of the present invention, the concentration C of the anti-TUBB3 autoantibodies, as well as all the threshold values, *x, y* and z, can be expressed in terms of concentration/volume or weight, or also in terms of absorbance at a suitable wavelength, when detection means comprising a spectrophotometric reading of the results are used.

Apparently, any methodology directly or indirectly determining the concentrations of anti-TUBB3 autoantibodies in serum may be used to implement the methods of the present invention and for the carrying out of the kits described hereinafter.

As mentioned above, therefore, the threshold value, as well as the concentration C of autoantibodies in the serum of analysed patients, can be expressed come values of absorbance at a suitable wavelength (depending on the detection system used).

Apparently, the same system of values will be used for the determining of the concentration of anti-TUBB3 autoantibodies in the serum and for the determining of the threshold values *x, y* and/or *z*.

Apparently, given a certain absorbance at a certain wavelength with a known protocol and detection system, it is possible to extrapolate from obtained absorbance values the corresponding concentrations of TUBB3 autoantibodies.

According to one embodiment of the present invention, the concentration C and the threshold value can therefore be expressed in terms of absorbance at a wavelength of between 500 and 400 nm, e.g., about: 500 nm, 490 nm, 480 nm, 470 nm, 460 nm, 450 nm, 440 nm, 430 nm, 420 nm, 410 nm, 400 nm. According to a specific embodiment, e.g. when using peroxidase in the detection system, said absorbance, said absorbance will be an absorbance at the wavelength of about 450 nanometres, A₄₅₀, and said threshold value x could be a threshold value of between 0.1 and 0.2 as absorbance at a 450nm wavelength (A₄₅₀). The technician in the field, by knowing the protocol used in detail, can easily extrapolate the corresponding threshold values in terms of antibody concentrations, therefore the object application also relates to the embodiment wherein said threshold values are expressed in terms of antibody concentration. The values reported in terms of absorbance have therefore precise equivalents in terms of antibody concentrations. According to one embodiment of the invention, therefore, the threshold value *x,* is A₄₅₀ of between 0.1 and 0.2.

According to a further embodiment, said threshold value *x* corresponds to about A₄₅₀ equal to 0.15 as cut-off between levels of anti-TUBB3 antibodies in healthy subjects (A₄₅₀ <0.15) and in diseased subjects (A₄₅₀ >0.15), with a calculated 90% sensitivity and 95.5% specificity percentage (AUC value equal to 0.97).

As mentioned above, all these values are quickly translatable by the technician in the field in terms of antibody concentrations, based on the data provided in the experimental section of the application.

Another object of the present invention relates to an *in vitro* method for the diagnosis of ovarian carcinoma and the prognosis of overall survival expectancy in case of presence of said ovarian carcinoma in a patient, comprising the following steps:
a) determining the concentration C of anti-TUBB3 autoantibodies in a serum sample of said patient;
b) comparing said concentration C of autoantibodies to a threshold value *x,* wherein a concentration of said autoantibodies higher than said threshold value *x* identifies the presence of ovarian carcinoma in said patient; and
c) when said patient is positive to ovarian carcinoma, comparing said concentration C of autoantibodies to a threshold value *y;* wherein an increasing difference between said concentration C and said second threshold value *y* corresponds to a decreasing overall survival expectancy of said patient.

According to this object of the invention, the threshold value *x* is as defined above.

According to the invention, the threshold value *y* is any one value of anti-TUBB3 autoantibodies serum concentration in a pool of samples collected from patients affected by ovarian carcinoma.

According to a specific embodiment, said threshold value *y* is equal to said threshold value *x.*

According to a further embodiment, said threshold value *y* is given by the median of the values of anti-TUBB3 autoantibodies serum concentration in a pool of samples collected from patients affected by ovarian carcinoma.

According to the present invention, a "high" concentration of anti-TUBB3 autoantibodies corresponds to a concentration above the median, whereas a "low" concentration of anti-TUBB3 autoantibodies corresponds to a concentration below or equal to the median.

In this case as well, the threshold value y could be expressed as value of absorbance at a suitable wavelength (depending on the detection system used).

Hence, according to one embodiment of the present invention, the concentration C and the different threshold values can therefore be expressed in terms of absorbance at a wavelength of between 500 and 400 nm, e.g. about: 500 nm, 490 nm, 480 nm, 470 nm, 460 nm, 450 nm, 440 nm, 430 nm, 420 nm, 410 nm, 400 nm. According to a specific embodiment, e.g. when using peroxidase in the detection system, said absorbance will be an absorbance at the wavelength of about 450 nanometers, A₄₅₀.

The above-described method, comprising also step c) enables the medical team to predict the OS of examined patients.

This method can therefore be introduced in a therapeutic method followed by a therapeutic step d) in which the patient is treated in a personalized manner based on the calculated OS. Therefore, for patients with a particularly poor OS, the medical team will more promptly carry out a surgical removal of ovarian carcinoma when possible, adjust the therapeutic regimen by modulating it depending on the known higher aggressiveness of the disease, carry out a stricter serial control over time in order to early identify disease progression, and an adequate counseling and psycho-oncological support for the patient and her family.

According to a specific embodiment, the above method, comprising steps a) b) and c), can also be carried out on patients for whom a diagnosis of ovarian carcinoma has already been made, and could therefore be carried out in a form limited to the sole steps a) and c) of prognosis of overall survival expectancy in a patient affected by ovarian carcinoma, comprising the following steps:
a) determining the concentration C of anti-TUBB3 autoantibodies in a serum sample of said patient;
c) comparing said concentration C of autoantibodies to a threshold value *y;* wherein an increasing difference between said concentration C and said second threshold value *y* corresponds to a decreasing overall survival expectancy of said patient.

The threshold value *x* in step a) and the threshold value *y* in step c) are as defined above. An *in vitro* method for the diagnosis of ovarian carcinoma and the predictability of drug resistance to taxol and/or derivatives thereof of said ovarian carcinoma, when present, in a patient, is disclosed. Said method comprises the following steps:
a) determining the concentration C of anti-TUBB3 autoantibodies in a serum sample of said patient;
b) comparing said concentration C of autoantibodies determined at step a) to a threshold value *x,* wherein a concentration of said autoantibodies higher than said threshold value identifies the presence of ovarian carcinoma in said patient; and
c) when said patient is positive to ovarian carcinoma, comparing said concentration of autoantibodies C to a threshold value z; wherein a concentration of said autoantibodies in said patient sample higher than said threshold value z is predictive of a resistance to taxol and/or derivatives thereof by said carcinoma.

The threshold value *x* in step b) is as previously defined in the present description. The threshold value z in step c) above is given by the median of the values of anti-TUBB3 autoantibodies serum concentration determined in a pool of samples collected from patients affected by ovarian carcinoma.

Alternatively, when carried out on patients for whom an ovarian carcinoma has already been diagnosed, the above-reported method may be limited to the sole predictability of drug resistance to taxol and/or derivatives thereof, excluding diagnostic step b). Also disclosed is an *in vitro* method predictive of drug resistance to taxol and/or derivatives thereof of ovarian carcinoma in a patient affected by ovarian carcinoma, comprising the following steps:
a) determining the concentration C of anti-TUBB3 autoantibodies in a serum sample of said patient;
c) comparing said concentration of autoantibodies C to a threshold value *z;*
wherein a concentration of said autoantibodies in said patient sample higher than said threshold value z is predictive of a resistance to taxol and/or derivatives thereof by said carcinoma.

The threshold value *x* in step b) is as previously defined in the present description. The threshold value z in step c) above is given by the median of the values of anti-TUBB3 autoantibodies serum concentration in a pool of samples collected from patients affected by ovarian carcinoma.
In this case as well, the threshold value z could be expressed as value of absorbance at a suitable wavelength (depending on the detection system used).
Hence, the concentration C and the threshold values can therefore be expressed in terms of absorbance at a wavelength of between 500 and 400 nm, e.g. about: 500 nm, 490 nm, 480 nm, 470 nm, 460 nm, 450 nm, 440 nm, 430 nm, 420 nm, 410 nm, 400 nm. According to a specific embodiment, e.g. when using peroxidase in the detection system, said absorbance will be an absorbance at the wavelength of about 450 nanometers, A₄₅₀.
In this case as well, the method comprising the step predictive of drug resistance to taxol and/or derivatives thereof, as well as the method for the prognosis of OS of the examined patient, can be introduced in a therapeutic method followed by a therapeutic step d) wherein the patient is treated in a personalized manner based on the result obtained in step c). Therefore, in cases in which a high probability for patients of exhibiting drug resistance to taxol and/or derivatives thereof is estimated, the medical team will more promptly carry out a removal of carcinoma when possible, an appropriate therapeutic regimen without taxol and/or derivatives thereof, a stricter control of disease progression, an adequate psychological support for the patient and her family.
In particular, the therapeutic regimen could be a therapeutic regimen comprising, e.g., pegylated liposomal doxorubicin which, in the MITO-2 randomized clinical trial, has recently shown a proven effectiveness in combination with carboplatin in the first-line treatment of ovarian carcinoma (Pignata S, Scambia G, Ferrandina G, et al. Carboplatin plus paclitaxel versus carboplatin plus pegylated liposomal doxorubicin as first-line treatment for patients with ovarian cancer: the MITO-2 randomized phase III trial. J Clin Oncol. 2011;29:3628-35) and represents the drug of election in the treatment of platinum-resistant recurrent ovarian carcinoma (Ferrandina G, Ludovisi M, Lorusso D, et al. Phase III trial of gemcitabine compared with pegylated liposomal doxorubicin in progressive or recurrent ovarian cancer. J Clin Oncol. 2008;26:890-6.); but also other chemotherapeutical agents indicated in the treatment of ovarian carcinoma in association with carboplatin, such as: gemcitabine and cyclophosphamide. Moreover, the medical team will avoid subjecting the patient to a useless chemotherapeutic regimen in which, therefore, the patient would experience only the disadvantages of chemotherapy, without benefiting from it.
Moreover, the method could concomitantly provide information on OS and on the expectancies of drug resistance, e.g., simply by *y*=*z* at step c).

The advantages deriving from the methods of the invention are immediately evident.

According to one embodiment, the determining in step a) of the above-reported methods is carried out with an ELISA test.
Such embodiment, besides being quick and easily repeatable, also has the advantage that the ELISA test ensures a semiquantitative photometric dosage of the analyte, obtained with continuous values of absorbance, and provides an objective result in comparison with the subjective (operator-dependent) and semiquantitative evaluation of immunohistochemistry methodologies used in the state of the art of diagnostics.

According to this embodiment, the threshold value *x* can be expressed in terms of serum concentration of the antibody, or in terms of absorbance, and can be, e.g., A₄₅₀ of between 0.1 and 0.2.
According to a specific embodiment of the above, said threshold value *x* can be equal to A₄₅₀ about 0.15, and represent the cut-off between levels of anti-TUBB3 antibodies in healthy (A₄₅₀ ≤0.15) and diseased (A₄₅₀ >0,15) subjects, with a calculated sensitivity percentage of 90%, and specificity percentage of 95.5% (AUC value equal to 0.97). Chemotherapeutical agents excluding taxol and/or derivatives thereof are disclosed for use in the treatment of ovarian carcinoma in patients selected according to an *in vitro* method for predicting chemoresistance to taxol and/or derivatives thereof by said carcinoma, comprising the following steps:
a) determining the concentration C of anti-TUBB3 autoantibodies in a serum sample of said patient;
c) comparing said concentration of autoantibodies C to a threshold value *z;*
wherein a concentration of said autoantibodies in said patient sample higher than said threshold value *z* is predictive of a resistance to taxol and/or derivatives thereof by said carcinoma;
wherein said treatment comprises the administration of said chemotherapeutical agents excluding taxol and/or derivatives thereof to patients exhibiting an anti-TUBB3 autoantibodies serum concentration higher than said threshold value y identified in step c) of said predictive method.

Specifically, the chemotherapeutical agents excluding taxol and/or derivatives thereof for the above-indicated use can be selected in the group comprising anthracyclines (e.g., pegylated liposomal doxorubicin, adriamycin, epirubicin), antimetabolites (e.g., gemcitabine), alkylating agents (e.g., cyclophosphamide and etoposide) or combinations thereof.

A further object of the invention is the use a kit for the diagnosis of ovarian cancer and/or for the prognosis of overall survival expectancy of patients affected by ovarian cancer and/or for prediction of chemoresistance to taxol in patients affected by ovarian cancer comprising one or more aliquots of reagents for the detection of anti TUBB3-autoantibodies in samples of serum and one or more control internal references for the quantification of these autoantibodies in the methods defined by the claims.

Since the threshold values *x, y,* or *z* might vary slightly depending on the cohort of patients analysed, and to make the test immediately readable, the diagnostic/prognostic/predictive kit used in the present invention is not merely a kit enabling the detection of anti-TUBB3 autoantibodies, but is a kit enabling to immediately establish the amount of anti-TUBB3 autoantibodies present in serum.

Thus, the kit enables an immediate reading of the results regardless of assigned threshold values that, as mentioned, can also vary simply according to the desired sensitivity and specificity.

In one embodiment, the kit is an ELISA kit.

Specifically, the ELISA kit used according to the present invention comprises at least two or more internal control references for the quantification of said anti-TUBB3 autoantibodies in the form of wells coated with known, increasing concentrations of TUBB3.

The bonding between TUBB3 placed in each well and the AUTOTUBB3 present in the serum, after addition of secondary antibody and chromogen determines the Aₙₘ values at a spectrophotometric reading (e.g., the A₄₅₀ range expected in the population studied in Table 1 is between 0.04 and 0.92)

In one embodiment of the invention, the internal control references are comprised of rows of wells coated with known, increasing concentrations of TUBB3 positioned in the ELISA plate so to provide detailed information on the concentration of anti-TUBB3 autoantibodies for each serum sample analysed (e.g., in rows or in columns).

A further object of the invention is the use of a kit comprising reagents for the quantitative detection of anti-TUBB3 autoantibodies in serum or of reagents for the quantitative detection of anti-TUBB3 autoantibodies in serum for carrying out any one of the above-described methods object of the present invention.

According to the description and the following examples, the term "low anti-TUBB3 autoantibodies" means a concentration of autoantibodies (expressed in terms of concentration or also in terms of absorbance) below the median or equal to the median of serum concentration of anti-TUBB3 autoantibodies measured in a sample of patients affected by ovarian carcinoma of reference (see, e.g., Table 1).

According to the description and the following examples, the term "high anti-TUBB3 autoantibodies" means a concentration of autoantibodies (expressed in terms of concentration or also in terms of absorbance) above the median of serum concentration of anti-TUBB3 autoantibodies measured in a sample of patients affected by ovarian carcinoma of reference.

The term "expression levels of anti-TUBB3 autoantibodies" can also correspond to the term "concentration of anti-TUBB3 autoantibodies", as such values are clearly correlated with each other.

The term TUBB3 represents human protein tubulin beta 3 or tubulin beta III, well-known in the literature NM_006086.3 (Reference sequence obtained from National Center for Biotechnology Information, NCBI, databank).

### EXAMPLES

**1)** Class III tubulin protein (TUBB3) is a protein normally expressed in neuronal cells and in testis, and not in epithelial cells, whereas it is overexpressed in many types of carcinomas. In one of their studies (not published), the Authors observed that TUBB3 overexpression in tumour tissue is correlated with the presence of anti-TUBB3 autoantibodies in serum. To single out the expression of these anti-TUBB3 autoantibodies, the Authors have used a simple, quick and inexpensive methodology such as ELISA, which does not require an extremely invasive intervention on the patient, as it only requires a collection of blood.

The ELISA methodology is direct, simple, does not need highly qualified professionals and uses inexpensive and easily available materials.

### TUBB3 recombinant protein production and purification in sf9 insect cells:

The Baculovirus system is widely used for massive production of recombinant proteins. The Inventors used INVITROGEN Bac-to-Bac Baculovirus Expression System. The pFastBac plasmid containing the gene of interest, cloned upstream of the sequence encoding 6 histidine (6xhis) (pFastHTA) is transformed into *E. Coli* DH10Bac™ competent cells. These contain a bacmid (baculoviral shuttle vector) with a mini-attTn7 target site and a helper plasmid. Once the expression plasmid pFast Bac (donor plasmid) is transformed into DH10Bac bacterial cells, the transposition between the mini Tn7 element present on the pFast Bac and the mini-attTn7 target site present on the bacmid occurs to generate a recombinant bacmid. The transposition occurs thanks to the presence of transposition proteins encoded by the helper. The recombinant bacmid DNA is isolated from DH10Bac positive cells and transfected into insect cells.

The insect cell lines most used for applying expression systems by baculoviral vectors (BEVS) are the Sf9 ones, deriving from ovarian tissue of *Spodoptera frugiperda* Lepidoptera. P1 Viral stock, initially obtained from Sf9 transfection, is a low-titer stock. It can be used to infect cells, so as to amplify the virus and obtain a high-titer P2 and P3 viral stock.

The suspension of Sf9 insect cells (1x 10⁶ cells/ml) is transferred into a Sf-900 II SFM 1X medium (Serum Free Medium Complete, GIBCO), with the addition of 0.1% Pluronic F-68 (GIBCO) in a plate stirrer. The Sf9 cells, at a concentration of 600x 10⁶, are infected with the P3 viral stock at an MOI of 0.5, with a viral titer of 2x10⁸ pfu/ml. At +72 hours, cells are harvested after centrifuging and washed with 1X PBS. The pellet thus obtained is suspended into a lysis buffer at pH6,5 with 1% Igepal CA-630 (SIGMA) and 1% of protease inhibitors. Lysed cells are incubated on ice for 30 minutes and ultrasonicated at 200-300W. Cells are subsequently centrifuged. To purify the protein, chromatography on polypropylene column (QIAGEN) is used. After a 16-hour incubation at 4 °C, the protein is eluted with an elution buffer at pH8.

The fraction thus obtained is analysed on SDS-PAGE acrylamide gel. Purified protein concentration is determined by BRADFORD method.

### Patients

The study included 49 ovarian carcinoma patients, admitted to the Gynecologic Oncology Unit of the *Università Cattolica* of Rome. Said patients gave their informed consent for collection of their clinical data and use of their biological samples (biopsies and serum samples) for research purposes.

Sera and tumour samples were taken, and the histopathological diagnosis was confirmed in all patients. Sera were collected at cancer diagnosis, prior to the patients' receiving chemotherapeutic treatment, whereas tumour tissue samples were collected at surgery, carried out before any treatment.

Serum samples were collected from healthy female donors that gave their informed consent for collection of their clinical data and use of their biological samples (serum samples) for research purposes.

The clinicopathological characteristics of all series are summarized in Table 1. Median age was 57 years (range: 25- 81). 39 cases (79.6%) were stage III-IV and 10 cases (20.4%) were stage I-II disease; 26 patients (53.1%) had carcinomatosis and 46 (93.9%) exhibited a high grade (G2-G3). cytoreduction to ≤1cm residual tumour was achieved in 26 (53%) of the cases, whereas cytoreduction to >1cm residual tumour was achieved in 23 (46.9%) of the cases.

**Table 1. Distribution of patients' clinicopathological characteristics and TUBB3 expression levels in tumour samples in accordance with anti-TUBB3 autoantibodies levels.**

| **Anti-beta tubulin III autoantibodies** | **All cases** | **Low TUBB3 AutoAb** | **High TUBB3 AutoAb** | ***p^{a} value*** |
|---|---|---|---|---|
| **Characteristics** | # (%) | # (%) | # (%) | |
| **All cases** | 49 | 25 (51.0) | 24 (49.0) | - |
| **TUBB3 AutoAb, median (range) A₄₅₀** | 0.31 (0.04-0.92) | 0.22 (0.04-0.31) | 0.41 (0.32-0.92) | - |
| **Median age (range) years^{b}** | 57 (25-81) | 55 (25-81) | 59 (40-71) | 0.384 |
| **FIGO Stage** | | | | |
| I-II | 10 (20.4) | 4 (16.0) | 6 (25.0) | |
| III-IV | 39 (79.6) | 21 (84.0) | 18 (75.0) | 0.335 |
| **Carcinomatosis** | | | | |
| Yes | 26 (53.1) | 12 (48.0) | 14 (58.3) | |
| No | 23 (46.9) | 13 (52.0) | 10 (41.7) | 0.469 |
| **Tumour histotype** | | | | |
| Serous | 39 (79.6) | 21 (84.0) | 18 (75.0) | |
| Endometrioid/Clear cells | 10 (20.4) | 4 (16.0) | 6 (25.0) | 0.335 |
| **Tumour grade** | | | | |
| G1 | 3 (6.1) | 3 (12.0) | 0 (0.0) | |
| G2-G3 | 46 (93.9) | 22 (88.0) | 24 (100.0) | 0.080 |
| **Residual tumour at 1^{st} surgery** | | | | |
| ≤ 1cm | 26 (53.1) | 14 (56.0) | 12 (50.0) | |
| > 1cm | 23 (46.9) | 11 (44.0) | 12 (50.0) | 0.447 |
| **TUBB3 IHC levels on tumour samples, median (range)** | 20 (0-90) | 10 (0-20) | 30 (10-90) | **0.001** |

| | | | | |
|---|---|---|---|---|
| *^{a}*Calculated with chi-square test *^{b}*Calculated with nonparametric Kruskal-Wallis test TUBB3-AutoAb: anti-beta III tubulin autoantibodies | | | | |

### Blood sample collection

Prior to surgery, 8ml whole blood were collected from each patient. Serum samples were obtained by using a serum-separating tube for the serum and stored at -80°C before the assay. Serum levels of anti-TUBB3 autoantibodies were measured by solid-phase chemoluminescent ELISA assay.

### ELISA (enzyme-linked immunosorbent assay):

Initially, a "coating" of the bottom of a 96-well plate was carried out with the TUBB3 recombinant protein produced and purified as described above, at a concentration of 0.25 µg/µl.

The plate was incubated overnight, at 4°C.

After having removed the "coating" solution and washed the plate with wash buffer (1X PBS 0.1% Tween20) the plate was incubated with a solution of 1X PBS - 0.1% Tween20 - 1% gelatin to block nonspecific binding sites for 1 hour at 37°C.

Subsequently, without washing the plate, 1 microliter of serum (1:100 dilution) of the 49 patients and of 50 healthy controls was dispensed in duplicate in a blocking buffer with 1X PBS- 0.1 % tween20, 1% gelatin, and incubated 1 hour at 37°C.

Following washing with 1X PBS- 0.1% Tween20 buffer, wells were incubated with 100ul of HRP-conjugated anti-human IgG secondary antibody for 1hour at 37°C.

After a washing with 1X PBS- 0.1%Tween20 buffer, 100ul chromogen were added into each well and left to react for 5-10 minutes at room temperature.

Possibly present anti-TUBB3 antibodies bind to the antigen coating the wells, and are therefore detected by the second antibody specific for human IgG, conjugated to horseradish peroxidase enzyme (Goat anti-human IgG-HRP, Catalog number 732581, Beckman Coulter Company).

The reaction was blocked with 100ul of "stop solution" (Sigma)

The absorbance value was determined spectrophotometrically with a plate reader at a 450-nm length.

The detection of the amount of bound Goat anti-human IgG-HRP (proportional to the amount of anti-TUBB3 antibodies present in each well) was then carried out by adding a substrate: the 3,3',5,5'-tetramethylbenzidine, HRP substrate (TMB). Under enzymatic action, TMB takes a blue colour. Change to blue is directly proportional to the amount of TUBB3-bound serum antibody. The chromatic intensity (absorbance) of the solution in the well is then measured on a spectrophotometer (Plate Reader; DAS srl) at a 450nm wavelength.

**2)** This technique can be used by all analysis laboratories, as it is simple, quick and inexpensive. For its carrying out, it employs a smaller number of professionals in comparison with the immunohistochemical technique commonly used to know the expression of factors involved in tumourigenesis or tumour progression, such as TUBB3.

In the study reported herein it was observed that patients with ovarian carcinoma express appreciable serum levels of anti-TUBB3 autoantibodies, unlike healthy donors taken as negative control (using ROC (Receiver Operating Characteristics) curve analysis, the sensitivity and specificity of the test proved to be respectively equal to 90% and 95.5% setting as dosage cut-off the A₄₅₀ value of 0.15. The calculated AUC value is equal to 0.97, therefore vouching for the high accuracy of the test). These results support the possible use of this methodology in early diagnosis of TUBB3-expressing ovarian tumour and, plausibly, in other tumour types where TUBB3 protein is over-expressed and leads to the production of autoantibodies. Moreover, this methodology can be used in a "prognostic kit" for patients with ovarian carcinoma or other tumours, given the involvement of the TUBB3 protein in tumour progression, or as "predictive kit" of response to chemotherapy, given TUBB3 role in the drug resistance (chemoresistance) phenomenon.

**3)** *A*: Absorbance; A₄₅₀: Absorbance at 450 nm; ELISA: enzyme-linked immunosorbent assay; pFastBac: bacterial plasmid; bacmid: viral vector; SDS PAGE: SDS-PolyAcrylamide Gel Electrophoresis; TIME COURSE: performed as transfection control; BRADFORD method: used for spectrophotometric measurement of protein concentration in a sample; pFastHTA: DNA fragment encoding for the TUBB3 protein;

To express analyte concentration in terms of units or nanograms per ml, it is necessary to make a reference standard. The immunoenzymatic dosage object of the present invention can avail itself of the possibility of titrating the anti-TUBB3 antibodies present in the serum by progressive dilution of the sera and subsequent expression of the concentration as antibody titer (i.e., the reverse of the highest dilution of the patient's serum that is dosable in the method object of the present invention), therefore becoming a semi-quantitative dosage (semi-quantitative assessment).

The Authors observed that a 1:100 dilution of patients' serum enabled in all cases to observe an absorbance value falling within the range of measurable signals. In fact, at such a dilution all absorbance values observed with a 450nm filter fell within the A₄₅₀ range of 0.025-2.5.

### Immunohistochemistry with TUBB3

Immunohistochemistry was carried out on 3-µm sections of paraffin-embedded tissue, mounted on slides coated with poly-lysine and dried at 37°C overnight.

After slides were deparaffined with xylene and rehydrated in a conventional way, endogenous peroxidase was blocked with 3% H₂O₂ in H₂O for 5 minutes.

Antigen detection procedure was carried out with a microwave oven, by heating 10mM citric acid, pH 6,0, twice for 10 min.

To reduce nonspecific binding, the sections were incubated with 20% normal goat serum for 30 minutes at room temperature.

TUBB3-expressing cells were identified after reacting for 1 hour at room temperature with human anti-TUBB3 monoclonal antibody (TUJ1; 1:300; COVANCE) in the presence of 20% normal goat serum.

TUBB3 detection was evaluated by reaction with EnVision-mouse+ System-HRP labeled polymer (DAKO, Carpinteria, CA, USA) for 30 minutes at room temperature.

Diaminobenzidine (DAB Substrate System, DAKO) was used as chromogen.

Sections were counterstained with hematoxylin, dehydrated, cleaned in xylene and mounted with Eukitt.

Negative controls were obtained by omitting the primary antibody. TUBB3-positive controls were represented by cerebral sections obtained from cerebral biopsies of healthy tissue.

Analysis of all tissue samples was carried out with normal light microscopy, under blind conditions, by two different operators of proven skills (GFZ; EM). The ratio of immuno-labeled tumour cells was measured with a low magnification (5X lenses) evaluating the entire tumour area.

### Western Blot Analysis

TUBB3 fusion protein (1.2 µg) was subjected to electrophoretic analysis on a 12% polyacrylamide-SDS gel and thereafter transferred on a PVDF membrane. A chemoluminescent molecular marker ("Magic Mark", Life Technologies) was used as run control. Membranes were blocked with dry skim milk and incubated with serum of patients affected by ovarian carcinoma or of healthy donors, diluted 1:100 in dry skim milk, overnight at 4°C under stirring. After 3 washings with tris-buffered-saline buffer with 0.1% Tween20 (TBST), membranes were incubated with horseradish peroxidase-conjugated goat anti-human IgG polyclonal antibody for 1 hour at room temperature (18°C). After 3 washings with TBST, specific signals on the membranes were detected by using the Pierce Enhanced Chemioluminescent Detection System.

Positive control was obtained by incubating the PVDF membrane with an anti-TUBB3 polyclonal antibody (Covance). Negative controls were obtained by incubating the PVDF membrane with the serum of healthy women donors.

### Statistical analysis

Differences between patients with high and low levels of anti-TUBB3 autoantibodies were analysed by using Pearson's exact chi-square test and the Kruskal-Wallis test as appropriate (Table 1).

Correlation between TUBB3 protein levels in tumour samples and in-serum presence of anti-TUBB3 autoantibodies was evaluated using Spearman's nonparametric test, which detected a significant correlation with a very favourable coefficient (equal to 0.804), between TUBB3 levels in tumour, evaluated by immunohistochemical analysis (y-axis) and in-serum anti-TUBB3 autoantibodies evaluated by ELISA method (x-axis) in the investigated cohort of 49 patients affected by ovarian carcinoma (Figure 3).

Overall survival (OS) and progression-free survival, or PFS, were calculated from the date of diagnosis to the date of progression/death or of the last visit. Medians and life tables were calculated by using the Kaplan-Meier method, and the Log-rank test was used merely to establish statistical significance (figures 4 and 5) (Kaplan FL, Meier P. Nonparametric estimation from incomplete observations. Am.J Stat Assoc 1958;53:457-81 and Mantel N. Evaluation of survival data and two new rank order statistics arising in its consideration. Cancer Chemother Rep 1966;50:163-70).

The clinical role of anti-TUBB3 autoantibodies expression levels and of other parameters analysed as continuous variable was established by Cox's proportional hazards model (Cox DR. Regression models and life tables. J R Stat Soc 1972;34:197-220), Figure 3, Table 2 below.

All statistical calculations were performed by using the Statistical Package for Social Sciences (Version 17.0, SPSS Inc., Chicago, IL, USA).

**Table 2. Univariate and multivariate analysis of clinicopathological parameters, and treatment details as overall survival prognosis.**

| | **Univariate analysis** | | **Multivariate analysis** | |
|---|---|---|---|---|
| **Variable** | ***X²*** | **p-value** | ***X²*** | **p-value** |
| **Age^{a}** | 2.606 | 0.102 | - | - |
| **FIGO Stage** | | | | |
| **I-II** | | | | |
| **IIIC-IV** | **3.741** | **0.035** | - | - |
| **Carcinomatosis** | | | | |
| **Yes** | | | | |
| **No** | **5.051** | **0.025** | 1.230 | 0.883 |
| **Tumour histotype** | | | | |
| **Serous** | | | | |
| **Endometrioid/Clear cells** | 0.601 | 0.614 | - | - |
| **Residual tumour at 1^{st} surgery** | | | | |
| **≤ 1cm** | | | | |
| **> 1cm** | **8.958** | **0. 001** | 2.462 | 0.177 |
| **Progression-free survival^{a}** | **13.210** | **0.001** | **9.249** | **0.001** |
| **Levels of circulating TUBB3-AutoAb-** | | | | |
| **High** | | | | |
| **Low** | **6.170** | **0.006** | **4.213** | **0.045** |
| **Levels of TUBB3 IHC in tumour samples** | | | | |
| **High** | | | | |
| **Low** | 0.662 | 0.416 | - | - |

| | | | | |
|---|---|---|---|---|
| ^{a}Considered as continuous variable | | | | |

Only variables exhibiting a *p<0.05* value in univariate analysis were included in the multivariate one
TUBB3-AutoAb: anti-tubulin beta III autoantibodies

### REFERENCE

Bansal D, Herbert F, Lim P, Deshpande P, Bécavin C, Guiyedi V, et al. IgG autoantibody to brain beta tubulin III associated with cytokine cluster-II discriminate cerebral malaria in central India. PLoS One. 2009 Dec 14;4(12):e8245
Defresne F, Bouzin C, Guilbaud C, Dieu M, Delaive E, Michiels C,et al. Differential influence of anticancer treatment and angiogenesis on the seric titer of autoantibody used as tumour and metastasis biomarker. Neoplasia; 2010.vol12 July, 565-570
Ferrandina G, Zannoni GF, Martinelli E, Paglia A, Gallotta V, et al. Class III beta-tubulin overexpression is a marker of poor clinical outcome in advanced ovarian cancer patients. Clin Cancer Res. 2006 May 1;12(9):2774-9
Grassadonia A, Tinari N, Natoli C, Yahalom G, lacobelli S. Circulating autoantibodies to LGALS3BP: a novel biomarker for cancer. Dis Markers. 2013;35(6):747-52)
Kavallaris M. Microtubules and resistance to tubulin-binding agents. Nat Rev Cancer. 2010 Mar; 10(3): 194-204
Mariani M, Karki R, Spennato M, Pandya D, He S, Andreoli M, Fiedler P , Ferlini C. Class III β-tubulin in normal and cancer tissues. Gene. 2015 Jun 1;563(2):109-14
Mozzetti S, Ferlini C, Concolino P, Filippetti F, Raspaglio G, Prislei S, et al. Class III beta tubulin overexpression is a prominent mechanism of paclitaxel resi stance in ovarian cancer. Clin Cancer Res. 2005 Jan 1;11(1):298-305

## Claims

1. An *in vitro* method for the diagnosis of ovarian carcinoma in a patient, comprising the following steps:
a) determining the concentration C of anti-TUBB3 autoantibodies in a serum sample of said patient;
b) comparing said concentration C of autoantibodies to a threshold value *x*, wherein a concentration of said autoantibodies higher than said threshold value identifies the presence of ovarian carcinoma in said patient.

2. An *in vitro* method for the diagnosis of ovarian carcinoma and the prognosis of overall survival expectancy in case of presence of said ovarian carcinoma in a patient, comprising the following steps:
a) determining the concentration C of anti-TUBB3 autoantibodies in a serum sample of said patient;
b) comparing said concentration C of autoantibodies to a threshold value *x,* wherein a concentration of said autoantibodies higher than said threshold value *x* identifies the presence of ovarian carcinoma in said patient; and
c) when said patient is positive to ovarian carcinoma, comparing said concentration C of autoantibodies to a threshold value *y;* wherein an increasing difference between said concentration C and said second threshold value *y* corresponds to a decreasing overall survival expectancy of said patient.

3. An *in vitro* method for the prognosis of overall survival expectancy in a patient affected by ovarian carcinoma, comprising the following steps:
a) determining the concentration C of anti-TUBB3 autoantibodies in a serum sample of said patient;
c) comparing said concentration C of autoantibodies to a threshold value *y;*
wherein an increasing difference between said concentration C and said threshold value *y* corresponds to a decreasing overall survival expectancy of said patient.

4. The method according to anyone of claims 1 to 3, wherein said determining in step a) is carried out with an ELISA test.

5. The method according to anyone of claims 1 to 4, wherein said threshold value *x* is calculated by comparing the distribution curves of serum concentrations of anti-TUBB3 autoantibodies of samples collected from healthy individuals and the distribution curves of serum concentrations of anti-TUBB3 autoantibodies of samples collected from patients affected by ovarian carcinoma, and using a ROC curve for defining said threshold value according to the desired specificity and sensitivity.

6. The method according to anyone of claims 1 to 4, wherein said serum concentrations of said autoantibodies and said threshold values *x* and/or *y* are expressed in terms of concentration/volume or concentration/weight.

7. The method according to anyone of claims 1 to 5, wherein said serum concentrations of said autoantibodies and said threshold values *x* and/or *y* are expressed in terms of absorbance at a suitable wavelength.

8. The method according to claim 7, wherein said wavelength is about 450 nm.

9. The method according to claim 8, wherein said threshold value x is between 0.1 and 0.2, or wherein said threshold value x is about 0.15.

10. The method according to anyone of claims 6 to 9, wherein said desired specificity is between 91.5% and 97.5%, or is higher than or equal to 95.5%

11. The method according to anyone of claims 6 to 9, wherein said desired sensitivity is between 87.5% and 91.5%, or is higher than or equal to 90%.

12. The method according to anyone of claims 2-11, wherein said threshold value *y* is any value indicating a serum concentration of anti-TUBB3 autoantibodies in pools of samples collected from patients affected by ovarian carcinoma

13. The method according to claim 12, wherein said threshold value *y* is equal to said threshold value *x.*

14. The method according to claim 12, wherein said threshold value *y* is given by the median of the anti-TUBB3 autoantibodies serum concentration in samples collected from patients affected by ovarian carcinoma.

15. Use of a kit comprising reagents for the detection of anti-TUBB3 autoantibodies or use of reagents for the detection of anti-TUBB3 autoantibodies for carrying out the method according to anyone of claims 1 to 14.

16. The use according to claim 15 wherein said kit comprises one or more aliquots of reagents for the detection of anti TUBB3-autoantibodies in serum samples and one or more control internal references for the quantification of these autoantibodies.

17. The use according to claim 16, wherein said kit is an ELISA kit and said one or more control internal references for the quantification of said anti TUBB3-autoantibodies are wells coated with known, increasing concentrations of TUBB3.

18. The use according to claim 17, wherein said wells coated with known, increasing concentrations of TUBB3 are positioned in the ELISA plate so to provide detailed information on the concentration of anti-TUBB3 autoantibodies for each serum sample analysed.

## Patentansprüche

1. *In-vitro*-Verfahren zur Diagnose von Ovarialkarzinom bei einer Patientin, das die folgenden Schritte umfasst:
a) Bestimmen der Konzentration C von anti-TUBB3-Autoantikörpern in einer Serumprobe der Patientin;
b) Vergleichen der Konzentration C von Autoantikörpern mit einem Schwellenwert *x,* wobei eine Konzentration der Autoantikörper, die höher als der Schwellenwert ist, das Vorliegen von Ovarialkarzinom in der Patientin anzeigt.

2. *In-vitro*-Verfahren zur Diagnose von Ovarialkarzinom und Prognose einer Gesamtüberlebenserwartung im Falle eines Vorliegens des Ovarialkarzinoms bei einer Patientin, das die folgenden Schritte umfasst:
a) Bestimmen der Konzentration C von anti-TUBB3-Autoantikörpern in einer Serumprobe der Patientin;
b) Vergleichen der Konzentration C von Autoantikörpern mit einem Schwellenwert *x,* wobei eine Konzentration der Autoantikörper, die höher als der Schwellenwert *x* ist, das Vorliegen von Ovarialkarzinom in der Patientin anzeigt; und
c) wenn die Patientin positiv auf Ovarialkarzinom getestet ist, Vergleichen der Konzentration C von Autoantikörpern mit einem Schwellenwert *y,* wobei eine zunehmende Differenz zwischen der Konzentration C und dem zweiten Schwellenwert *y* einer abnehmenden Gesamtüberlebenserwartung der Patientin entspricht.

3. *In-vitro*-Verfahren zur Prognose einer Gesamtüberlebenserwartung bei einer Patientin mit Ovarialkarzinom, das die folgenden Schritte umfasst:
a) Bestimmen der Konzentration C von anti-TUBB3-Autoantikörpern in einer Serumprobe der Patientin;
c) Vergleichen der Konzentration C von Autoantikörpern mit einem Schwellenwert *y,* wobei eine zunehmende Differenz zwischen der Konzentration C und dem zweiten Schwellenwert *y* einer abnehmenden Gesamtüberlebenserwartung der Patientin entspricht.

4. Verfahren gemäß einem der Ansprüche 1 bis 3, wobei das Bestimmen in Schritt a) mit einem ELISA-Test ausgeführt wird.

5. Verfahren gemäß einem der Ansprüche 1 bis 4, wobei der Schwellenwert *x* durch Vergleichen der Verteilungskurven von Serumkonzentrationen von anti-TUBB3-Autoantikörpern von Proben, die von gesunden Personen gewonnen werden, mit den Verteilungskurven von Serumkonzentrationen von anti-TUBB3-Autoantikörpern von Proben, die von Patientinnen mit Ovarialkarzinom gewonnen werden, berechnet wird, wobei eine ROC-Kurve verwendet wird, um den Schwellenwert hinsichtlich der gewünschten Spezifität und Sensitivität zu definieren.

6. Verfahren gemäß einem der Ansprüche 1 bis 4, wobei die Serumkonzentrationen der Autoantikörper und die Schwellenwerte *x* und/oder *y* als Konzentrationen in Bezug auf Volumen oder Gewicht ausgedrückt werden.

7. Verfahren gemäß einem der Ansprüche 1 bis 5, wobei die Serumkonzentrationen der Autoantikörper und die Schwellenwerte x und/oder *y* als Absorbierung bei einer geeigneten Wellenlänge ausgedrückt werden.

8. Verfahren gemäß Anspruch 7, wobei die Wellenlänge ungefähr 450 nm beträgt.

9. Verfahren gemäß Anspruch 8, wobei der Schwellenwert x zwischen 0,1 und 0,2 liegt, oder der Schwellenwert *x* ungefähr 0,15 beträgt.

10. Verfahren gemäß einem der Ansprüche 6 bis 9, wobei die gewünschte Spezifität zwischen 91,5 % und 97,5 % liegt, oder höher als oder gleich 95,5 % ist.

11. Verfahren gemäß einem der Ansprüche 6 bis 9, wobei die gewünschte Sensitivität zwischen 87,5 % und 91,5 % liegt, oder höher als oder gleich 90 % ist.

12. Verfahren gemäß einem der Ansprüche 2 - 11, wobei der Schwellenwert *y* ein beliebiger Wert ist, der eine Serumkonzentration von anti-TUBB3-Autoantikörpern in Pools von Proben, die von Patientinnen mit Ovarialkarzinom gewonnen werden, anzeigt.

13. Verfahren gemäß Anspruch 12, wobei der Schwellenwert *y* gleich dem Schwellenwert x ist.

14. Verfahren gemäß Anspruch 12, wobei der Schwellenwert *y* durch den Median der anti-TUBB3-Autoantikörperserumkonzentration in Proben, die von Patientinnen mit Ovarialkarzinom gewonnen worden sind, bestimmt wird.

15. Verwendung eines Kits, das Reagenzien zur Detektion von anti-TUBB3-Autoantikörpern umfasst, oder Verwendung von Reagenzien zur Detektion von anti-TUBB3-Autoantikörpern zur Ausführung des Verfahrens gemäß einem der Ansprüche 1 bis 14.

16. Verwendung gemäß Anspruch 15, wobei das Kit umfasst: ein oder mehrere Aliquots von Reagenzien zur Detektion von anti-TUBB3-Autoantikörpern in Serumproben und eine oder mehrere interne Kontrollreferenzen zur Quantifizierung dieser Autoantikörper.

17. Verwendung gemäß Anspruch 16, wobei das Kit ein ELISA-Kit ist und die eine oder mehreren internen Kontrollreferenzen zur Quantifizierung der anti-TUBB3-Autoantikörper Wells sind, die mit bekannten, steigenden Konzentrationen von TUBB3 beschichtet sind.

18. Verwendung gemäß Anspruch 17, wobei die mit bekannten, steigenden Konzentrationen von TUBB3 beschichteten Wells in der ELISA-Platte so angeordnet sind, dass sie detaillierte Informationen über die Konzentration von anti-TUBB3-Autoantikörpern für jede analysierte Serumprobe zur Verfügung stellen.

## Revendications

1. Procédé *in vitro* pour le diagnostic d'un carcinome ovarien chez une patiente, comprenant les étapes suivantes :
a) détermination de la concentration C d'auto-anticorps anti-TUBB3 dans un échantillon de sérum de ladite patiente ;
b) comparaison de ladite concentration C d'auto-anticorps avec une valeur de seuil *x,*
dans lequel une concentration desdits auto-anticorps supérieure à ladite valeur de seuil identifie la présence d'un carcinome ovarien chez ladite patiente.

2. Procédé *in vitro* pour le diagnostic d'un carcinome ovarien et le pronostic d'une espérance de vie globale dans le cas de la présence dudit carcinome ovarien chez une patiente, comprenant les étapes suivantes :
a) détermination de la concentration C d'auto-anticorps anti-TUBB3 dans un échantillon de sérum de ladite patiente ;
b) comparaison de ladite concentration C d'auto-anticorps avec une valeur de seuil *x,*
dans lequel une concentration desdits auto-anticorps supérieure à ladite valeur de seuil *x* identifie la présence d'un carcinome ovarien chez ladite patiente ; et
c) quand ladite patiente est positive à un carcinome ovarien, la comparaison de ladite concentration C d'auto-anticorps avec une valeur de seuil *y* ;
dans lequel une augmentation de la différence entre ladite concentration C et ladite deuxième valeur de seuil *y* correspond à une diminution de l'espérance de vie globale de ladite patiente.

3. Procédé *in vitro* pour le pronostic d'une espérance de vie globale d'une patiente affectée par un carcinome ovarien, comprenant les étapes suivantes :
a) détermination de la concentration C d'auto-anticorps anti-TUBB3 dans un échantillon de sérum de ladite patiente ;
c) la comparaison de ladite concentration C d'auto-anticorps avec une valeur de seuil *y* ;
dans lequel une augmentation de la différence entre ladite concentration C et ladite deuxième valeur de seuil *y* correspond à une diminution de l'espérance de vie globale de ladite patiente.

4. Procédé selon l'une quelconque des revendications 1 à 3, dans lequel ladite détermination dans l'étape a) est effectuée avec un test ELISA.

5. Procédé selon l'une quelconque des revendications 1 à 4, dans lequel ladite valeur de seuil *x* est calculée par comparaison des courbes de distribution de concentrations sériques d'auto-anticorps anti-TUBB3 d'échantillons collectés auprès d'individus en bonne santé, et des courbes de distribution de concentrations sériques d'auto-anticorps anti-TUBB3 d'échantillons collectés auprès de patientes affectées par un carcinome ovarien, et utilisation d'une courbe ROC pour définir ladite valeur de seuil en fonction de la spécificité et de la sensibilité souhaitées.

6. Procédé selon l'une quelconque des revendications 1 à 4, dans lequel lesdites concentrations sériques desdits auto-anticorps et lesdites valeurs de seuil *x* et/ou *y* sont exprimées en termes de concentration/volume ou de concentration/poids.

7. Procédé selon l'une quelconque des revendications 1 à 5, dans lequel lesdites concentrations sériques desdits auto-anticorps et lesdites valeurs de seuil *x* et/ou *y* sont exprimées en termes d'absorbance à une longueur d'onde convenable.

8. Procédé selon la revendication 7, dans lequel ladite longueur d'onde est d'environ 450 nm.

9. Procédé selon la revendication 8, dans lequel ladite valeur de seuil *x* est comprise entre 0,1 et 0,2, ou dans lequel ladite valeur de seuil *x* est d'environ 0,15.

10. Procédé selon l'une quelconque des revendications 6 à 9, dans lequel ladite spécificité souhaitée est comprise entre 91,5 % et 97,5 %, ou est supérieure ou égale à 95,5 %.

11. Procédé selon l'une quelconque des revendications 6 à 9, dans lequel ladite sensibilité souhaitée est comprise entre 87,5 % et 91,5 %, ou est supérieure ou égale à 90 %.

12. Procédé selon l'une quelconque des revendications 2 à 11, dans lequel ladite valeur de seuil *y* est n'importe quelle valeur indiquant une concentration sérique d'auto-anticorps anti-TUBB3 dans des groupes d'échantillons collectés auprès de patientes affectées par un carcinome ovarien.

13. Procédé selon la revendication 12, dans lequel ladite valeur de seuil y est égale à ladite valeur de seuil *x.*

14. Procédé selon la revendication 12, dans lequel ladite valeur de seuil *y* est donnée par la médiane de la concentration sérique d'auto-anticorps anti-TUBB3 dans des échantillons collectés auprès de patientes affectées par un carcinome ovarien.

15. Utilisation d'une trousse comprenant des réactifs pour la détection d'auto-anticorps anti-TUBB3 ou utilisation de réactifs pour la détection d'auto-anticorps anti-TUBB3 pour la mise en œuvre du procédé de l'une quelconque des revendications 1 à 14.

16. Utilisation selon la revendication 15, dans laquelle ladite trousse comprend une ou plusieurs aliquotes de réactifs pour la détection d'auto-anticorps anti-TUBB3 dans des échantillons de sérum et une ou plusieurs références internes témoins pour la quantification de ces auto-anticorps.

17. Utilisation selon la revendication 16, dans laquelle ladite trousse est une trousse ELISA et lesdites une ou plusieurs références internes témoins pour la quantification desdits auto-anticorps anti-TUBB3 sont des puits revêtus de concentrations croissantes connues de TUBB3.

18. Utilisation selon la revendication 17, dans laquelle lesdits puits revêtus de concentrations croissantes connues de TUBB3 sont positionnés dans la plaque d'ELISA de façon à offrir des informations détaillées sur la concentration d'auto-anticorps anti-TUBB3 pour chaque échantillon de sérum analysé.
